# EUROPEAN PATENT SPECIFICATION

(11) **EP 1 545 695 B1**
(45) Date of publication and mention of the grant of the patent: **20.08.2008**
(21) Application number: 03798626.2
(22) Date of filing: 17.09.2003
(51) Int. Cl.: A61N 1/08, A61N 1/378

(54) **DETECTION OF IMPLANTED WIRELESS ENERGY RECEIVING DEVICE**
NACHWEIS EINER IMPLANTIERTEN DRAHTLOSEN ENERGIE-EMPFANGSVORRICHTUNG
DETECTION D'UN DISPOSITIF DE RECEPTION D'ENERGIE SANS FIL IMPLANTE

(30) Priority: 25.09.2002 US 253897
(43) Date of publication of application: 29.06.2005
(73) Proprietor: Potencia Medical AG, 6341 Baar (CH)
(72) Inventor: FORSELL, Peter, CH-6300 Zug (CH)
(74) Representative: Strandin, Heléne
(86) International application number: PCT/SE2003/001458
(87) International publication number: WO 2004/028623

(56) References cited:
- WO-A1-01/37926
- US-A- 5 314 453
- US-A- 5 630 836
- US-A- 6 088 619
- US-B1- 6 473 652

## Description

The present invention relates to an apparatus and method for detecting a wireless energy receiving device subcutaneously implanted in a patient to enable accurate positioning of an exterior wireless energy transmission device.

In new generations of energy consuming implants wireless energy transmission may be used for supply of energy to such implants. To optimise the transfer efficiency of the wireless energy it is important to locate the wireless energy receiver, typically subcutaneously implanted in the patient, in order to be able to put an exterior energy transmission device close to the implanted energy receiving device. Document US-A-5 314 453 shows an arrangement for localising an implanted receiver coil and aligning a transmitter antenna coil with the former. The arrangement includes a magnet placed concentrically within the implanted receiver coil and a reed switch as magnetic field sensing device placed within the transmitter antenna coil.

The object of the present invention is to provide an inexpensive apparatus for accurate detection of a wireless energy receiving device subcutaneously implanted in a patient, and further to provide an apparatus with parts to be implanted that are relatively small.

This object is obtained by an apparatus as defined in claim 1, comprising a magnetic device adapted to emit a local magnetic field, and a magnetic detector adapted to detect the local magnetic field emitted by the magnetic device. The magnetic device is designed to be subcutaneously implanted in the patient at the implanted energy receiving device, and the magnetic detector is movable externally along the patient's body to establish an energy transmission position at the patient's skin where the local magnetic field emitted by the magnetic device is detected by the magnetic detector. Alternatively, the magnetic detector is designed to be subcutaneously implanted and the magnetic device is movable along the patient's body to establish the energy transmission position at the patient's skin where the local magnetic field emitted by the magnetic device is detected by the magnetic detector. As a result, the wireless energy transmission device can be put in the established energy transmission position, in order to efficiently transmit wireless energy to the implanted energy receiving device.

Thus, the present invention provides an easy way of detecting the position of the wireless energy receiving device subcutaneously implanted in a patient, which enables accurate positioning of the wireless energy transmission device outside the patient's body for efficient transmission of wireless energy to the implanted energy receiving device by using magnetism. Typical applications for such an energy receiving device are for energizing an implant in the patient, for example for treating reflux disease, obesity, anal or urinary incontinence, or impotence. The apparatus of the invention may conveniently be used for detecting an implanted energy receiving device coupled to such implants.

In accordance with a main first embodiment of the invention, the magnetic device is designed to be subcutaneously implanted in the patient at the implanted energy receiving device to emit the local magnetic field through a portion of the patient's skin adjacent to the energy receiving device, and the magnetic detector is movable externally along the patient's body to establish the energy transmission position where the local magnetic field is detected by the magnetic detector. Suitably, the magnetic detector is adapted to establish the energy transmission position at least substantially in front of the subcutaneously implanted energy receiving device to enable a most efficient energy transmission. The magnetic detector may be adapted to emit a sound when detecting the local magnetic field. Alternatively, the magnetic detector may be provided with at least one diode adapted to emit light when the detector detects the local magnetic field, or be provided with a display adapted to indicate when the detector detects the local magnetic field.

In accordance with a second embodiment of the invention, the magnetic detector is designed to be subcutaneously implanted in the patient at the implanted energy receiving device, and the magnetic device is adapted to emit the local magnetic field through the patient's skin from outside the patient's body and is movable externally along the patient's body to establish the energy transmission position where the local magnetic field is detected by the implanted magnetic detector. Also in this second embodiment the magnetic device is suitably adapted to establish the energy transmission position at least substantially in front of the subcutaneously implanted energy receiving device. In its simplest form, the implanted magnetic detector may be adapted to emit a sound when detecting the local magnetic field. In a more sophisticated form, a sender may be implantable in the patient's body and be capable of sending information about the magnetic detector to outside the patient's body, as the magnetic detector detects the local magnetic field emitted by the magnetic device from outside the patient's body. For example, the implanted sender may send RF signals that inform when the implanted detector detects the local magnetic field, whereby an accurate energy transmission position at the patient's skin can be established. The accurate energy transmission position may be directly or indirectly correlated to the intensity of magnetism detected by the magnetic detector.

The magnetic device may be a solenoid or a permanent magnet, which is sending out a magnetic field.

In any of the above embodiments the magnetic detector includes a semiconductor circuit, preferably in the form of at least one Hall-element. By using one or more Hall-elements, a special type of semiconductor known in the art, it is easy to locate the centre of the magnetic field emitted by the magnetic device. The magnetic detector suitably comprises several Hall-elements grouped around a central point in a triangular or square configuration. For example, three Hall-elements may be arranged at the corners of an equilateral triangle. An important advantage is that the Hall-elements are able to detect even a weak magnetic field emitted from the magnetic device.

The apparatus of the present invention is useful in a method of detecting a wireless energy receiving device subcutaneously implanted in a patient. The method comprises providing a magnetic device capable of emitting a local magnetic field through the patient's skin, providing a magnetic detector adapted to detect the local magnetic field emitted by the magnetic device, subcutaneously implanting the magnetic device or magnetic detector in the patient at the implanted energy receiving device, moving the magnetic detector or magnetic device externally along the patient's body, and establishing an energy transmission position at the patient's skin where the local magnetic field emitted by the magnetic device is detected by the magnetic detector. Then, the energy transmission device can be positioned in the established energy transmission position where the local magnetic field has been detected to efficiently transmit wireless energy to the subcutaneously implanted energy receiving device.

Where the magnetic detector is subcutaneously implanted and the magnetic device is moved along the patient's body, the method may further comprise implanting a sender and using the sender to send information to outside the patient's body confirming when the implanted magnetic detector detects the local magnetic field emitted by the exterior magnetic device.

The apparatus of the present invention may also be used in a surgical method for treating a patient having a disease, comprising the steps of: insufflating the patient's abdomen with gas; placing at least two laparoscopic trocars in the patient's body; implanting an operable implant designed for treating reflux disease, urinary incontinence, impotence, anal incontinence or obesity in the abdomen by using surgical instruments through the trocars; subcutaneously implanting a wireless energy receiving device for supplying energy for use in the operation of the implant and a magnetic device at the energy receiving device for emitting a local magnetic field through the adjacent skin portion of the patient; post-operatively moving an exterior magnetic detector along the patient's body to establish an energy transmission position in which the local magnetic field emitted by the implanted magnetic device is detected by the magnetic detector; bringing a wireless energy transmission device to the established energy transmission position; and transmitting wireless energy from the wireless energy transmission device through the patient's skin to the implanted wireless energy receiving device.

As an alternative, the surgical method may comprise subcutaneously implanting a magnetic detector at the energy receiving device and post-operatively moving an exterior magnetic device emitting a local magnetic field along the patient's body to establish the energy transmission position in which the local magnetic field emitted by the exterior magnetic device is detected by the implanted magnetic detector.

The skilled person should appreciate that the detection technique described above is simple, inexpensive and very accurate, and could be used for several different implants in combination with wireless energy transmission.

The invention is described in more detail in the following with reference to the accompanying drawings, in which
Fig.1 shows a connection diagram for a magnetic detector of the apparatus according to the present invention,
Fig.2 schematically illustrates in a diagram the output of the magnetic detector positioned in front of a magnetic device of the apparatus of the invention,
Fig. 3 is a schematic view of an embodiment where the magnetic device is subcutaneously implanted in a patient, and the magnetic detector is movable externally along the patient's body,
Fig. 4 is a schematic view of an embodiment where the magnetic detector is subcutaneously implanted in the patient and the magnetic device is movable externally along the patient's body,
Fig. 5 is a schematic view of a hydraulically adjustable constriction device designed for treating reflux disease, urine incontinence, anal incontinence or obesity, and
Fig. 6 illustrates an embodiment according to the present invention using Hall-elements as the magnetic detecting device.

Referring to the drawing figures, like reference numerals designate identical or corresponding elements throughout the several figures.

### DETAILED DESCRIPTION OF THE INVENTION

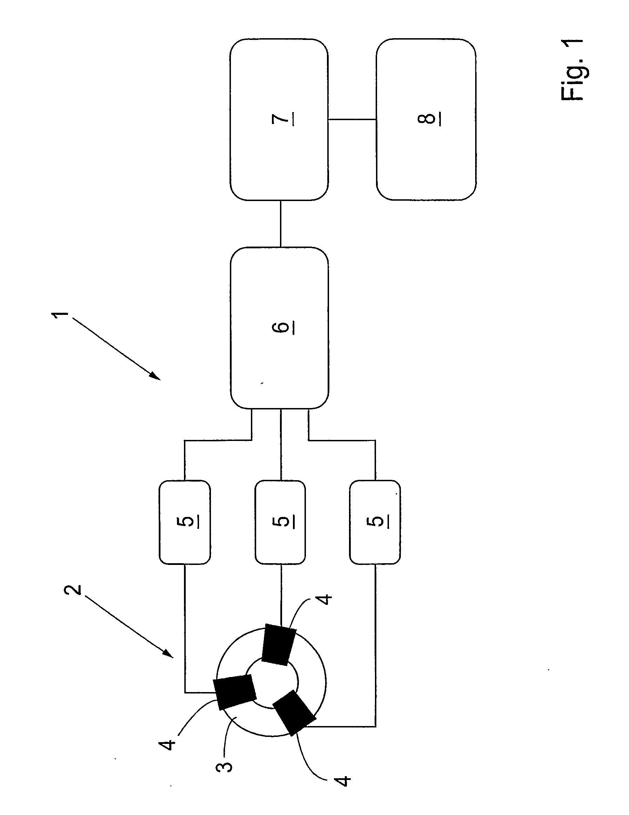

Fig. 1 shows a connection circuit 1 for a magnetic detector 2 of the apparatus according to the present invention. A magnetic device in the form of a ring-magnet 3, which can be a solenoid or a permanent magnet, is implanted in a patient's body. Located outside the body and positioned in front of the implanted ring-magnet 3 is magnetic detector 2, which includes three linear magnetic field sensors 4 (such as Hall-elements or the like) grouped in a triangular configuration. Sensors 4 are connected to signal-conditioning amplifiers 5, which in turn, are connected to an A/D-converter 6. A microprocessor 7 is connected to A/D-converter 6. To visually display the output signals of sensors 4, a display-device 8 is connected to microprocessor 7.

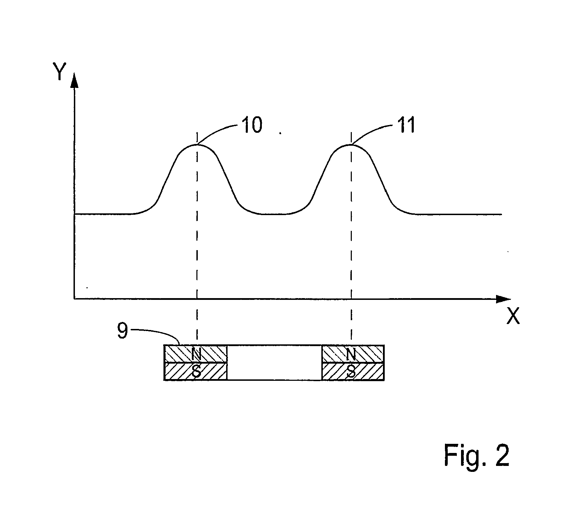

The graph shown in Fig. 2 illustrates, in principle, how the information obtained by detector 2 can be presented. On the X-axis in the graph is the position of detector 2 relative to the magnetic device. On the Y-axis is the combined output of sensors 4 of detector 2. Thus, the graph of Fig. 2 shows the position "X" of detector 2 relative to the magnetic device as a function of detector 2's output "Y". To illustrate this method of detecting, a magnetic device in the form of a ring-magnet 9 is shown relative to the graph of Figure 2. Ring-magnet 9 is shown in cross-section to show the positions of its magnetic north pole N and south pole S, respectively. Fig. 2 depicts the case where magnetic detector 2 (not shown in Fig. 2) has been centred in front of ring-magnet 9 and where all of the sensors 4 produce a maximum output, which is shown as peaks 10,11 in the graph of Fig. 2. Sensors 4 are connected (e.g., by connection circuit 1 shown in Fig. 1) to display device 8, which may display the graph shown in Fig. 2, or alternatively, a numeral result from the measurements taken by sensors 4.

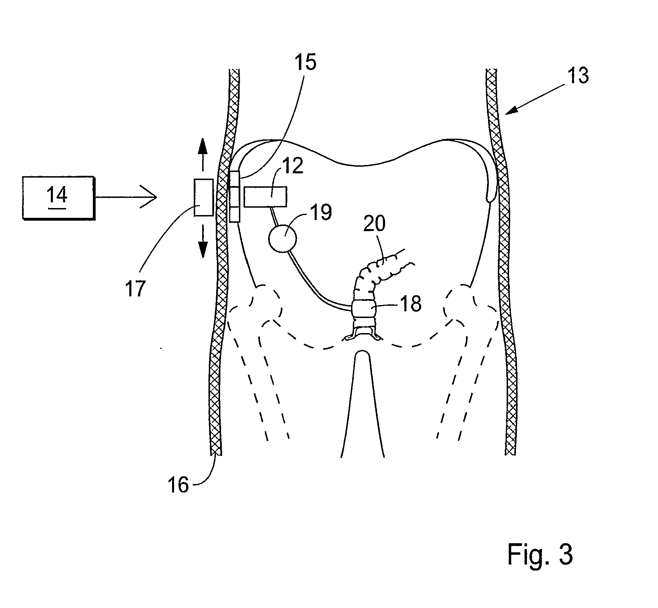

Fig. 3 shows an embodiment of the apparatus of the present invention for detecting a wireless energy receiving device 12 subcutaneously implanted in a patient 13 suffering from anal incontinence to enable accurate positioning of a separate wireless energy transmission device 14 outside patient 13's body for the transmission of wireless energy to energy receiving device 12. An operation device 19 is adapted to operate an implanted artificial sphincter 18 applied to the patient's rectum 20. Energy receiving device 12 powers operation device 19 with energy received from the energy transmission device 14. The implanted energy receiving device 12 may be of the type that transforms the received energy into electrical pulses. In this case, a sender may be implanted in the patient for sending feedback information on the number of electrical pulses that have been provided by the energy receiving device. The apparatus also includes a magnetic device 15 subcutaneously implanted in patient 13 close to the energy receiving device 12. Magnetic device 15 emits a local magnetic field extending through a portion of patient 13's skin 16 adjacent to energy receiving device 12. The apparatus further includes an external, separate magnetic detector 17 that may be manually moved along the patient 13's body to establish an energy transmission position at the patient's skin where the local magnetic field emitted by magnetic device 15 is detected by magnetic detector 17. When this energy transmission position has been established, energy transmission device 14 can be located in the same position to efficiently transmit wireless energy to implanted energy receiving device 12.

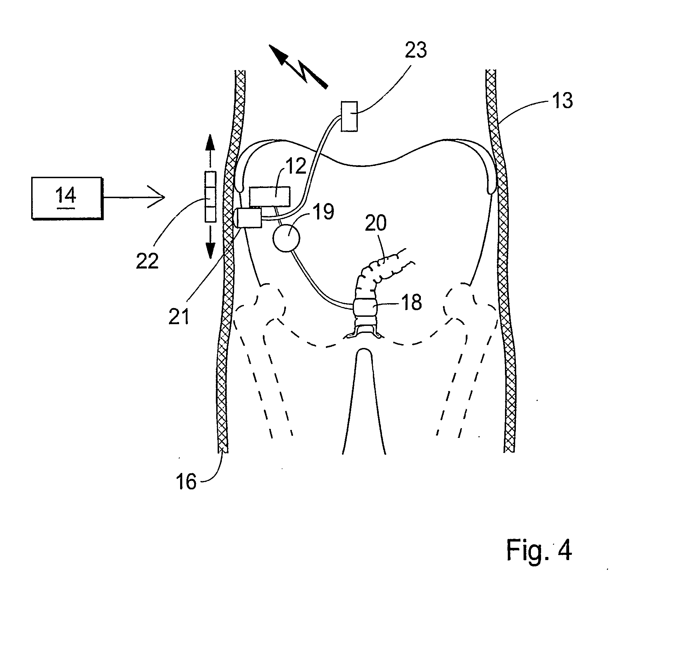

Fig. 4 shows an embodiment of the invention identical to the embodiment according to Fig. 3, except that a magnetic detector 21 is subcutaneously implanted in patient 13 at energy receiving device 12 and an external separate magnetic device 22 that emits a local magnetic field through patient's 13 skin 16 is provided. Magnetic device 22 may be manually moved externally along the patient 13's body to establish an energy transmission position at the patient's skin where the local magnetic field emitted by magnetic device 22 is detected by the implanted magnetic detector 21. A sender 23 is implanted in patient 13 and sends information about the status of magnetic detector 21. Thus, when magnetic detector 21 detects the local magnetic field emitted by external magnetic device 22, sender 23 sends information confirming that magnetic device 22 is in a proper position for energy transmission. When this energy transmission position has been established, energy transmission device 14 can be placed in the same position to efficiently transmit wireless energy to the implanted energy receiving device 12.

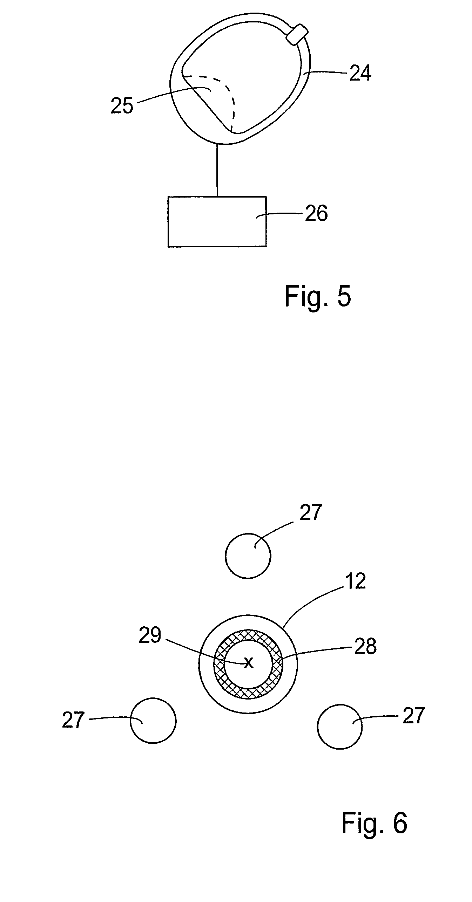

Fig. 5 shows an example of the artificial sphincter 18 shown in Figs. 3 and 4. Sphincter 18 includes a hydraulically adjustable constriction device 24 to be applied around the patient's rectum (not shown in Fig. 5). Constriction device 24 has a cavity 25 which can be inflated by supplying hydraulic fluid thereto, to close the rectum, and be deflated by withdrawing hydraulic fluid therefrom, to open the rectum. A hydraulic operation device 26 for operating constriction device 24 is powered with energy from implanted energy receiving device 12. This type of constriction device may also be used as an artificial sphincter for treating patient's suffering from heartburn and reflux disease or urinary incontinence, when combined with the apparatus of the present invention. Furthermore, constriction device 24 may be used for forming an adjustable stoma opening in the stomach or esophagus of an obese patient to treat obesity or for restricting the penile exit blood flow to treat an impotent patient, when combined with the apparatus of the invention.

Fig. 6 shows an advantageous design of the embodiment shown in Fig. 3, in which the external magnetic detector 17 includes three symmetrically arranged Hall-elements 27 which are grouped around a central point in a triangular configuration. The magnetic device 15 is implanted and includes a ring-shaped magnet 28 surrounding the centre 29 of the implanted energy receiving device 12. When magnetic detector 17 is moved to a position in which Hall-elements 27 are placed symmetrically above and around ring-shaped magnet 28, as illustrated in Fig. 6, magnetic detector 17 detects a maximum intensity of the magnetic field emitted by the implanted magnet 28, whereby the most accurate position where the energy transmission device 14 should be placed is established. As an alternative, the design described above may be practised in the embodiment shown in Fig. 4. Thus, the implanted magnetic detector 21 may include the three Hall-elements 27 and the external magnetic device 22 may include the ring-shaped magnet 28.

Although the present invention has been described in terms of a particular embodiment and process, it is not intended that the invention be limited to that embodiment. Modifications of the embodiments of the invention will be apparent to those skilled in the art. The scope of the invention is defined by the claims that follow.

## Claims

1. An apparatus for detecting a wireless energy receiving device (12) adapted to be subcutaneously implanted in a patient (13) to enable accurate positioning: of a wireless energy transmission device (14) outside the patient's body for transmission of wireless energy to the implanted energy receiving device, the apparatus comprising: a magnetic device (15 ; 22) adapted to emit a local magnetic field, and a magnetic detector (17 ; 21) adapted to detect the local magnetic field emitted by the magnetic device, wherein the magnetic device (15) or magnetic detector (21) is designed to be subcutaneously implanted in the patient at the implanted energy receiving device (12), and the magnetic detector (17) or magnetic device (22) is movable externally along the patient's body to establish an energy transmission position at the patient's skin (16) where the local magnetic field emitted by the magnetic device is detected by the magnetic detector, whereby the wireless energy transmission device (14) can be put in the established energy transmission position, in order to efficiently transmit wireless energy to the implanted energy receiving device,
**characterised in that,**
the Magnetic detector (17 ; 21) comprises several magnetic field sensors (4) grouped around a central point in a triangular_ or square-configuration.

2. An apparatus according to claim 1, wherein the magnetic device (15) is designed to be subcutaneously implanted in the patient at the implanted energy receiving device (12) to emit the local magnetic field through a portion of the patient's skin (16) adjacent to the energy receiving device, and the magnetic detector (17) is movable externally along the patient's body to establish the energy transmission position where the local magnetic field is detected by the magnetic detector.

3. An apparatus according to claim 2, wherein the
magnetic detector is adapted to establish the energy transmission position at least substantially in front of the subcutaneously implanted energy receiving device.

4. An apparatus according to claim 1, wherein the magnetic detector (21) is designed to be subcutaneously implanted in the patient at the implanted energy receiving device (12), and the magnetic device (22) is adapted to emit the local magnetic field through the patient's skin (16) from outside the patient's body and is movable externally along the patient's body to establish the energy transmission position where the local magnetic field is detected by the implanted magnetic detector.

5. An apparatus according to claim 4, wherein the magnetic device is adapted to establish the energy transmission position at least substantially in front of the subcutaneously implanted energy receiving device.

6. An apparatus according to claim 4 or 5, further comprising a sender (23) implantable in the patient's body and capable of sending information about the magnetic detector (21) to outside the patient's body, as the magnetic detector detects the local magnetic field emitted by the magnetic device (22) from outside the patient's body.

7. An apparatus according to any one of claims 1-6, wherein the magnetic detector is adapted to emit a sound when detecting the local magnetic field.

8. An apparatus according to claim 2 or 3, wherein the magnetic detector is provided with at least one diode adapted to emit light when the detector detects the local magnetic field.

9. An apparatus according to claim 2 or 3, wherein the magnetic detector is provided with a display adapted to indicate when the detector detects the local magnetic field.

10. An apparatus according to any one of claims 1-9, wherein the magnetic device (15; 22) is a solenoid or a permanent magnet.

11. An apparatus according to any one of claims 1-10, wherein the magnetic detector comprises a semiconductor circuit.

12. An apparatus according to claim 11, wherein the semiconductor circuit of the magnetic detector comprises at least one Hall-element (27).

13. An apparatus according to claim 12, wherein the magnetic detector (17 ; 21) comprises several Hall-elements (27) grouped around a central point in said triangular or square- configuration.

14. Use of the apparatus according to any one of claims 1-13 for detecting a wireless energy receiving device, which is subcutaneously implanted in a patient and which energizes an implant in the patient for treating reflux disease, obesity, anal or urinary incontinence, or impotence.

## Patentansprüche

1. Vorrichtung zum Nachweis einer drahtlosen Energieempfangsvorrichtung (12), die geeignet ist, in einen Patienten (13) subkutan implantiert zu werden, um eine akkurate Positionierung einer drahtlosen Energieübertragungsvorrichtung (14) außerhalb des Körpers des Patienten zur Übertragung von drahtloser Energie an die implantierte Energieempfangsvorrichtung zu ermöglichen, wobei die Vorrichtung umfasst:
eine magnetische Vorrichtung (15, 22), die geeignet ist, ein lokales Magnetfeld zu emittieren, und einen magnetischen Detektor (17, 21), der geeignet ist, das von der magnetischen Vorrichtung emittierte lokale Magnetfeld zu erfassen, wobei die magnetische Vorrichtung (15) oder der magnetische Detektor (21) konzipiert ist, um an der implantierten Energieempfangsvorrichtung (12) subkutan in den Patienten implantiert zu werden, und der magnetische Detektor (17) oder die magnetische Vorrichtung (22) extern entlang des Körpers des Patienten beweglich ist, um eine Energieübertragungsposition auf der Haut (16) des Patienten zu bestimmen, wo das von der magnetischen Vorrichtung emittierte lokale Magnetfeld von dem magnetischen Detektor erfasst wird, wobei die drahtlose Energieübertragungsvorrichtung (14) an die bestimmte Energieübertragungsposition gestellt werden kann, um drahtlose Energie effizient an die implantierte Energie-Empfgangsvorrichtung zu übertragen,
**dadurch gekennzeichnet, dass**
der magnetische Detektor (17, 21) mehrere Magnetfeldsensoren (4) umfasst, die in einer dreieckigen oder quadratischen Konfiguration um einen mittleren Punkt gruppiert sind.

2. Vorrichtung nach Anspruch 1, wobei die magnetische Vorrichtung (15) konzipiert ist, um an der implantierten Energie-Empfangsvorrichtung (12) subkutan in den Patienten implantiert zu werden, um das lokale Magnetfeld durch einen Teil der Haut (16) des Patienten neben der Energieempfangsvorrichtung zu emittieren, und der magnetische Detektor (17) extern entlang des Körpers des Patienten beweglich ist, um die Energieübertragungsposition zu bestimmen, wo das lokale Magnetfeld vom magnetischen Detektor erfasst wird.

3. Vorrichtung nach Anspruch 2, wobei der magnetische Detektor geeignet ist, um die Energieübertragungsposition mindestens im Wesentlichen vor der subkutan implantierten Energieempfangsvorrichtung zu bestimmen.

4. Vorrichtung nach Anspruch 1, wobei der magnetische Detektor (21) konzipiert ist, um an der implantierten Energieempfangsvorrichtung (12) subkutan in den Patienten implantiert zu werden, und die magnetische Vorrichtung (22) geeignet ist, um das lokale Magnetfeld durch die Haut (16) des Patienten von außerhalb des Körpers des Patienten zu emittieren, und extern entlang des Körpers des Patienten beweglich ist, um die Energieübertragungsposition zu bestimmen, wo das lokale Magnetfeld vom implantierten magnetischen Detektor erfasst wird.

5. Vorrichtung nach Anspruch 4, wobei die magnetische Vorrichtung geeignet ist, die Energieübertragungsposition zumindest im Wesentlichen vor der subkutan implantierten Energieempfangsvorrichtung zu bestimmen.

6. Vorrichtung nach Anspruch 4 oder 5, weiter einen Sender (23) umfassend, der in den Körper des Patienten implantierbar ist und in der Lage ist, Informationen über den magnetischen Detektor (21) aus dem Körper des Patienten nach außen zu senden, wenn der magnetische Detektor das von der magnetischen Vorrichtung (22) von außerhalb des Körpers des Patienten emittierte lokale Magnetfeld erfasst.

7. Vorrichtung nach einem der Ansprüche 1 bis 6, wobei der magnetische Detektor geeignet ist, um ein Geräusch auszusenden, wenn das lokale Magnetfeld erfasst wird.

8. Vorrichtung nach Anspruch 2 oder 3, wobei der magnetische Detektor mit mindestens einer Diode versehen ist, die geeignet ist, Licht auszusenden, wenn der Detektor das lokale Magnetfeld erfasst.

9. Vorrichtung nach Anspruch 2 oder 3, wobei der magnetische Detektor mit einer Anzeige versehen ist, die geeignet ist, um anzuzeigen, wenn der Detektor das lokale Magnetfeld erfasst.

10. Vorrichtung nach einem der Ansprüche 1 bis 9, wobei die magnetische Vorrichtung (15, 22) ein Solenoid oder ein Permanentmagnet ist.

11. Vorrichtung nach einem der Ansprüche 1 bis 10, wobei der magnetische Detektor einen Halbleiterschaltkreis umfasst.

12. Vorrichtung nach Anspruch 11, wobei der Halbleiterschaltkreis des magnetischen Detektors mindestens ein Hall-Element (27) umfasst.

13. Vorrichtung nach Anspruch 12, wobei der magnetische Detektor (17, 21) mehrere Hall-Elemente (27) umfasst, die in der dreieckigen oder quadratischen Konfiguration um einen mittleren Punkt gruppiert sind.

14. Verwendung der Vorrichtung nach einem der Ansprüche 1 bis 13 zum Erfassen einer drahtlosen Energieempfangsvorrichtung, welche subkutan in einen Patienten implantiert ist und welche ein Implantat im Patienten mit Strom versorgt, um eine Reflux-Erkrankung, Fettsucht, Darm- oder Harninkontinenz oder Impotenz zu behandeln.

## Revendications

1. Appareil de détection d'un dispositif de réception d'énergie sans fil (12) conçu pour être implanté de manière sous-cutanée chez un patient (13) afin de permettre le positionnement précis d'un dispositif de transmission d'énergie sans fil (14) à l'extérieur du corps du patient en vue de la transmission d'énergie sans fil au dispositif de réception d'énergie implanté, l'appareil comprenant : un dispositif magnétique (15 ; 22) conçu pour émettre un champ magnétique local et un détecteur magnétique (17 ; 21) conçu pour détecter le champ magnétique local émis par le dispositif magnétique, où le dispositif magnétique (15) ou le détecteur magnétique (21) est conçu pour être implanté de manière sous-cutanée chez le patient au niveau du dispositif de réception d'énergie implanté (12), et le détecteur magnétique (17) ou le dispositif magnétique (22) est mobile à l'extérieur le long du corps du patient pour établir une position de transmission d'énergie au niveau de la peau du patient (16) où le champ magnétique local émis par le dispositif magnétique est détecté par le détecteur magnétique, grâce à quoi le dispositif de transmission d'énergie sans fil (14) peut être placé dans la position de transmission d'énergie établie, afin de transmettre efficacement de l'énergie sans fil au dispositif de réception d'énergie implanté,
**caractérisé en ce que**
le détecteur magnétique (17 ; 21) comprend plusieurs capteurs de champ magnétique (4) regroupés autour d'un point central en une configuration en triangle ou en carré.

2. Appareil selon la revendication 1, dans lequel le dispositif magnétique (15) est conçu pour être implanté de manière sous-cutanée chez le patient au niveau du dispositif de réception d'énergie implanté (12) pour émettre le champ magnétique local à travers une partie de la peau du patient (16) adjacente au dispositif de réception d'énergie, et le détecteur magnétique (17) est mobile à l'extérieur le long du corps du patient pour établir la position de transmission d'énergie à l'endroit où le champ magnétique local est détecté par le détecteur magnétique.

3. Appareil selon la revendication 2, dans lequel le détecteur magnétique est conçu pour établir la position de transmission d'énergie au moins sensiblement en face du dispositif de réception d'énergie implanté de manière sous-cutanée.

4. Appareil selon la revendication 1, dans lequel le détecteur magnétique .(21) est conçu pour être implanté de manière sous-cutanée chez le patient au niveau du dispositif de réception d'énergie implanté (12) et le dispositif magnétique (22) est conçu pour émettre le champ magnétique local à travers la peau du patient (16) depuis l'extérieur du corps du patient et est mobile à l'extérieur le long du corps du patient pour établir la position de transmission d'énergie à l'endroit où le champ magnétique local est détecté par le détecteur magnétique implanté.

5. Appareil selon la revendication 4, dans lequel le dispositif magnétique est conçu pour établir la position de transmission d'énergie au moins sensiblement en face du dispositif de réception d'énergie implanté de manière sous-cutanée.

6. Appareil selon la revendication 4 ou 5, comprenant en outre un émetteur (23) pouvant être implanté dans le corps du patient et capable d'envoyer des informations relatives au détecteur magnétique (21) à l'extérieur du corps du patient, lorsque le détecteur magnétique détecte le champ magnétique local émis par le dispositif magnétique (22) depuis l'extérieur du corps du patient.

7. Appareil selon l'une quelconque des revendications 1 à 6, dans lequel le détecteur magnétique est conçu pour émettre un son lors de la détection du champ magnétique local.

8. Appareil selon la revendication 2 ou 3, dans lequel le détecteur magnétique est muni au moins d'une diode conçue pour émettre de la lumière lorsque le détecteur détecte le champ magnétique local.

9. Appareil selon la revendication 2 ou 3, dans lequel le détecteur magnétique est muni d'un dispositif d'affichage conçu pour indiquer l'instant auquel le détecteur détecte le champ magnétique local.

10. Appareil selon l'une quelconque des revendications 1 à 9, dans lequel le dispositif magnétique (15 ; 22) est un solénoïde ou un aimant permanent.

11. Appareil selon l'une quelconque des revendications 1 à 10, dans lequel le détecteur magnétique comprend un circuit à semiconducteur.

12. Appareil selon la revendication 11, dans lequel le circuit à semiconducteur du détecteur magnétique comprend au moins un élément à effet Hall (27).

13. Appareil selon la revendication 12, dans lequel le détecteur magnétique (17 ; 21) comprend plusieurs éléments à effet Hall (27) regroupés autour d'un point central dans ladite configuration en triangle ou en carré.

14. Utilisation de l'appareil selon l'une quelconque des revendications 1 à 13 destiné à détecter un dispositif de réception d'énergie sans fil, qui est implanté de manière sous-cutanée chez un patient et qui alimente en énergie un implant chez le patient pour traiter une maladie de reflux, l'obésité, une incontinence anale ou urinaire ou l'impuissance.
